# FASCICULE DE BREVET EUROPEEN

(11) **EP 1 004 897 B1**
(45) Date de publication et mention de la délivrance du brevet: **22.08.2001**
(21) Numéro de dépôt: 99402935.3
(22) Date de dépôt: 25.11.1999
(51) Int. Cl.: G01T 1/164

(54) **Gamma-caméra convertible**
Konvertierbare Gammakamera
Convertible gamma-camera

(30) Priorité: 27.11.1998 FR 9815000
(43) Date de publication de la demande: 31.05.2000
(73) Titulaire: Sopha Médical Vision International, 75008 Paris (FR)
(72) Inventeur: Treillet, Jean, 95000 Cergy (FR); Pare, Christian, 95000 Cergy (FR)
(74) Mandataire: Schmit, Christian Norbert Marie

(56) Documents cités:
- EP-A- 0 517 600
- WO-A-96/30781

## Description

La présente invention a pour objet une gamma-caméra convertible, de type tunnel, de préférence avec deux couronnes, et à usage universel. Le but de l'invention est de rendre plus rationnelle la conception d'une telle gamma-caméra afin qu'elle puisse servir à des usages multiples.

On connaît dans le domaine de la médecine nucléaire les gamma-caméras du type PET ou du type SPECT (Positon Emission Tomography, tomographie par émission en coïncidence, Single Photon Emission Computed Tomography, tomographie construite à partir d'émissions de photons individuels). Dans le premier cas il s'agit de détecter l'apparition de phénomènes d'émission nucléaire en coïncidence sur deux détecteurs opposés et en vis-à-vis. Dans l'autre cas, il s'agit de détecter des émissions nucléaires émises dans au moins une direction. L'invention s'applique aussi bien à l'une qu'à l'autre de ces deux techniques.

Le principe de ces phénomènes est le suivant. On injecte dans un corps à examiner, celui d'un patient, un marqueur radiologique, par exemple le plus généralement du technétium, quelquefois du thallium, ou d'autres marqueurs. En fonction d'une charge métabolique, ces marqueurs vont se fixer de manière préférentielle dans certains organes du corps du patient. Par la densité de cette fixation, ils révèlent alors l'état fonctionnel de l'organe dans lequel ils se fixent. Ces marqueurs émettent au cours de leur métabolisation des rayons gamma. Ces rayons gamma sont détectés par un détecteur.

Un tel détecteur comporte sur une face d'entrée un collimateur dont le rôle est d'assurer l'acquisition d'une image en projection. Il est toutefois connu de disposer des collimateurs à trous inclinés ou à trous focalisés. Les rayons gamma qui traversent le collimateur aboutissent ensuite dans un scintillateur dont le rôle est de transformer chaque rayon gamma en une scintillation lumineuse. Cette scintillation lumineuse est détectable par un ensemble de tubes photomultiplicateurs disposés en aval du scintillateur. Les tubes photomultiplicateurs détectent les scintillations et délivrent en correspondance des signaux électriques dont l'amplitude et la forme sont révélatrices de la nature et du lieu de l'événement nucléaire détecté. On traite ces signaux, notamment dans des matrices de barycentration, pour constituer une image révélatrice de cette activité nucléaire.

Plusieurs types d'examens sont effectués. On distingue notamment d'une part les tomographies et d'autre part les images corps entier. Dans une tomographie on acquiert une image en projection pour une incidence donnée du détecteur par rapport au corps du patient. Puis on acquiert une autre image en projection pour une autre incidence, décalée de quelques degrés par rapport à la première. Ainsi de suite, on acquiert une multitude d'images en projection dont on extrait par des algorithmes de reconstruction d'image (du même type que ceux utilisés en tomodensitométrie) une image en trois dimensions de l'organe étudié. Les images en projection sont acquises lentement. Typiquement la durée d'acquisition d'une image en projection oscille entre cinq secondes pour les plus rapides, jusqu'à deux minutes pour les images les plus fines. Dans la demande de brevet européen EP-A-0 517 600, il a été expliqué que pour accélérer l'acquisition d'images en tomographie il était préférable d'utiliser deux détecteurs, et par ailleurs de placer ces détecteurs à 90° l'un de l'autre autour du corps du patient.

Un autre examen concerne les images dites corps entier. Pour acquérir une telle image, on déplace le détecteur en translation le long du corps du patient alors qu'il est allongé sur le dos. On prélève essentiellement avec ce type d'examen une image d'angiographie des jambes du patient, notamment pour reconnaître des problèmes d'insuffisance de vascularisation. De préférence également dans ce cas, pour augmenter la qualité de l'image, on utilise un deuxième détecteur placé sous le corps du patient. Le patient passe alors en translation entre ces deux détecteurs. Avec certaines machines, le patient est immobile, la machine se déplace par rapport à lui.

D'autres examens sont pratiqués telles les cardiographies d'effort. Pour ces autres examens, un patient se soumet à un effort physique en même temps que des images sont prises.

Les différents types d'examens pratiqués rendent nécessaire une grande mobilité des détecteurs les uns par rapport aux autres. Les machines conçues pour les mener à bien sont des machines de deux types : des machines tunnels ou des machines à statif ouvert dites à bras. Dans une machine tunnel le patient passe dans un tunnel dont les parois supportent mécaniquement les détecteurs. Dans les machines à bras, un statif maintient deux bras supportant chacun un détecteur et entre lesquels le patient est présenté. On connaît par exemple par la demande de brevet PCT/US95/13180 une machine mixte tunnel et à bras. Le problème présenté par ces différentes machines est celui de leur complexité de fabrication. En effet la technologie de détection, notamment celle des collimateurs, impose des détecteurs lourds. En pratique le poids de ces détecteurs peut être de l'ordre de 300 ou 400 kilos. Compte tenu des variétés de mise en place de ces détecteurs autour du corps du patient, et de la précision requise de l'ordre du millimètre à chaque fois, les bâtis qui supportent ces machines sont massifs. Ils le sont d'ailleurs d'autant plus que dans les hôpitaux ou dans les cabinets médicaux où ils sont installés souvent le sol sur lequel ils reposent n'est pas parfait. Dans ce cas ces bâtis doivent être capables de s'affranchir de ces problèmes de sol.

Par ailleurs pour passer d'un examen de type tomographique à un examen de type corps entier, les machines tunnel et les machines à bras ont des avantages et des inconvénients différents. Dans une machine tunnel, il suffit de faire passer le lit qui supporte le patient au travers du tunnel. Dans une machine à bras, il est nécessaire de tourner le lit qui supporte le patient de 90° par rapport à la machine avant de pouvoir pratiquer l'examen corps entier. Cet inconvénient est toutefois contrebalancé avec cette machine par la possibilité d'utiliser, en lieu et place du lit d'examen, le lit dans lequel le patient repose dans l'hôpital. Pour les patients grabataires ou intransportables hors de leur lit, il suffit de remplacer le lit de la machine par le lit du patient.

Pour diverses raisons les machines tunnels sont cependant préférées.

Un problème à résoudre est donc de concevoir une machine tunnel qui puisse permettre des angiographies de patients quand ces patients sont laissés sur leur lit d'hôpital, et quand ils ne sont pas couchés sur le lit d'examen. Ce problème est partiellement résolu par la machine mixte évoquée ci-dessus. Cependant celle-ci s'avère de conception onéreuse puisqu'elle comporte tous les mécanismes des deux machines. En outre, pour les examens au cours desquels le patient est assis ou debout face à la machine, ou bien en travail dans une machine d'effort, la présentation frontale des détecteurs proposée par une telle machine rend ces examens peu pratiques à mener. L'encombrement du piétement de cette machine mixte ainsi que la présence de son propre lit gênent pour ces examens.

Dans l'invention on a résolu ce problème en choisissant une machine tunnel. Cette machine tunnel n'est cependant pas une machine compliquée. Le problème de complexité y est résolu en munissant un support d'un détecteur d'un bras rabattable. Le bras rabattable, ou rabat, ainsi réalisé peut occuper deux positions préférentielles. Une première position du rabat dans le support présente le détecteur en regard du tunnel de la machine. Dans cette position, on peut pratiquer aussi bien des tomographies que des examens corps entier. Ces derniers sont alors pratiqués sur des patients susceptibles de venir se placer dans le lit de la machine. Dans l'autre position, un seul détecteur est déporté par le bras rabattable. Il est déporté à côté de la machine. On dispose alors, parallèlement à ce tunnel de la machine, de beaucoup d'espace pour y placer une chaise d'examen, une machine de travail, ou un lit d'un patient intransportable.

Dans ce cas, dans l'invention ces derniers examens ne seront pratiqués qu'avec un seul détecteur. En conséquence pour obtenir une qualité suffisante des images, la pose dans chaque image devra être suffisamment longue. Cependant ceci n'est pas pénalisant parce que ces examens sont relativement peu fréquents et que, de ce point de vue, l'invention réalise un bon compromis entre le coût de la machine et son efficacité réelle. Par ailleurs, le système de rabattage du bras est très simple. De ce fait, il ne provoque pas de coût supplémentaire important.

L'invention a donc pour objet une gamma-caméra de type tunnel avec au moins une couronne tournant autour d'un axe de rotation passant dans un tunnel, cette couronne portant un détecteur par l'intermédiaire d'un support, caractérisée en ce que ce support comporte un rabat lié mécaniquement d'une part à la couronne et d'autre part au détecteur, ce rabat étant muni d'une articulation avec un axe décalé par rapport au détecteur et pouvant occuper au moins deux positions angulaires stables par rapport à cet axe décalé, une première position angulaire permettant de présenter le détecteur qu'il porte en regard du tunnel, une deuxième position angulaire permettant de le présenter à côté du tunnel.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :
- Figure 1: une représentation d'une gamma-caméra selon l'invention avec un bras rabattable qui est dans une position telle que le détecteur soit situé à côté du tunnel d'examen de la machine ;
- Figures 2a à 2c : différentes possibilités d'utilisation de la machine de l'invention selon les types d'examen pratiqués ;
- Figure 3 : une représentation d'une utilisation de la machine de l'invention, ainsi que des particularités de réalisation de l'articulation du bras rabattable.

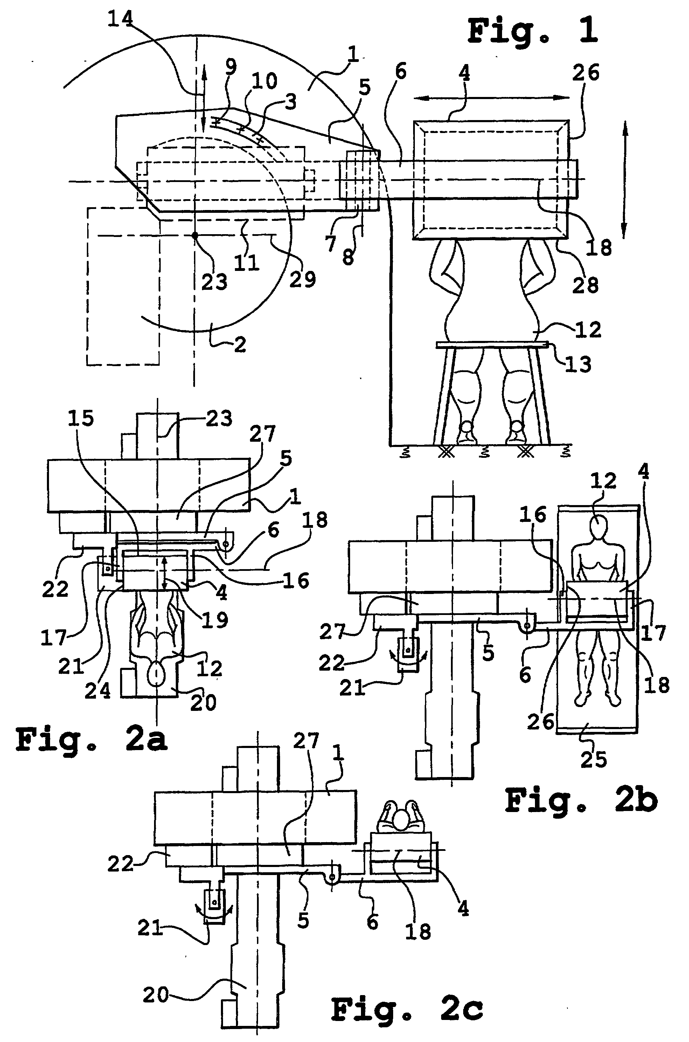

La figure 1 montre une gamma-caméra selon l'invention. Celle-ci comporte un bâti 1 percé d'un tunnel 2 et supportant en rotation une couronne 3. Dans la pratique le bâti 1 supporte deux couronnes. Chaque couronne porte un détecteur. La couronne 3 porte ainsi un détecteur 4 par l'intermédiaire d'un support 5. Le support 5 a l'allure d'une platine plane verticale. On verra plus loin qu'il est lui-même mobilisable en translations verticale et horizontale dans un plan vertical par rapport à la couronne 3. Le support 5 de l'invention présente la particularité essentielle de comporter un rabat 6. Le rabat 6, ou bras rabattable, est fixé à la platine 5 par l'intermédiaire d'une articulation 7 permettant le rabattement du rabat 6 autour d'un axe 8.

Dans une première position angulaire, le rabat 6 est rabattu sur la platine 5 emmenée à la couronne 3. Lorsque le rabat 6 occupe la première position angulaire, le détecteur 4 montré par le contour en pointillés 11 est présenté en regard du tunnel 2 de la machine 1. Dans une deuxième position angulaire, le détecteur 4 est présenté à côté du tunnel. Ceci est obtenu par le fait que l'axe 8 est décalé latéralement par rapport au détecteur 4.

La fixation de la platine 5 à la couronne 3 est schématiquement montrée avec des vis 9 et 10. Dans la pratique une ou plusieurs autres platines sont mécaniquement liées à la couronne 3, la platine 5 étant mobile par rapport à cette ou ces autres platines. Dans la pratique entre la couronne 3 et la platine 5 est ainsi aménagé un mécanisme de translation permettant à la platine 5 de se déplacer dans un plan parallèle à la face d'entrée de la machine 1. Ce déplacement comporte des translations latérales, verticales et mixtes.

Dans le type d'examen montré, un patient 12 est assis sur un siège 13 avec son dos et ses épaules faisant face à une face de détection (invisible ici) du détecteur 4. On remarquera que le fait de placer avec le rabat 6 le détecteur à côté du bâti 1 permet de disposer d'un espace important pour y placer le siège 13, ou une machine de travail. En utilisant la translation verticale 14 qui met en mouvement la platine 5, on provoque un déplacement équivalent du détecteur 4 le long du dos du patient 12. Eventuellement celui-ci peut être orienté avec son ventre plaqué contre la face de détection du détecteur 4.

Le passage d'une position angulaire du rabat 6 à l'autre est très facile. Il suffit de placer l'axe 8 verticalement. Dans ce cas on peut tourner le rabat 6 à la main, après l'avoir déverrouillé de la platine 5, pour qu'il occupe la position montrée. Les deux positions sont stables. Des mécanismes de verrouillage sont prévus pour maintenir le rabat dans l'une et l'autre positions.

Les figures 2a à 2c montrent des particularités d'utilisation d'une machine à double couronne. Sur la figure 2a on distingue la platine 5 et le rabat 6, ce dernier occupant une première position dans laquelle il est plaqué contre la platine 5. Le rabat 6 comporte un étrier 15 muni de deux jambes respectivement 16 et 17. Des paliers non représentés mais fixés aux jambes 16 et 17, à leurs extrémités, permettent de maintenir en orientation autour d'un axe (ici horizontal) 18 le détecteur 4. Une petite dimension 19 du détecteur 4 s'inscrit facilement dans la profondeur de l'étrier 15. Le détecteur 4 a une forme plutôt allongée, rectangulaire. L'axe 18 passe par la moitié du petit côté 19. Une grande dimension du détecteur 4 se loge entre les jambes 16 et 17 de l'étrier 15. De cette façon cet étrier 15 peut faire subir au détecteur 4 une rotation de 90° par rapport à la position horizontale représentée.

Sur la figure 2a, un patient 12 est couché sur un lit d'examen 20 de la machine. Il peut y subir un examen corps entier ou une tomographie. Pour cette dernière on utilise de préférence un deuxième détecteur 21 porté par un deuxième support 22, et présenté vers le patient, à 90° de la présentation du détecteur 4. Pour l'acquisition de la tomographie, la platine 5 et le support 22 tournent ensemble dans le bâti 1 selon un axe de rotation 23 passant par le centre du tunnel 2 de la machine. L'axe 18 est orthogonal à l'axe 23 dans les deux positions angulaires du détecteur 4. Par leurs déplacements en translation (verticaux et horizontaux), les supports 5 et 22 peuvent être approchés l'un de l'autre de telle façon qu'une arête 24 commune aux deux détecteurs 4 et 21 vient au contact. Pour un examen d'angiographie ou plus généralement de type corps entier, il suffit de faire tourner le support 22 seul autour du corps du patient 12 pour qu'il vienne se placer en dessous du lit 20. On peut alors acquérir une telle image avec les deux détecteurs simultanément ce qui en améliore la qualité. Dans ces deux examens le lit 20 peut glisser par rapport au bâti 1 le long du tunnel 2.

Sur la figure 2b, le rabat 6 occupe la deuxième position. Les jambes 16 et 17 des étriers, sensiblement horizontales, sont présentées au-dessus du corps du patient 12 celui-ci étant couché sur son lit d'hôpital 25 et non pas sur un lit d'examen. Comme sur la figure 2a, la face active du détecteur 4 reste orientée vers le bas. Par comparaison avec la figure 1, on constate que d'une figure à l'autre le détecteur 4 a basculé autour de l'axe 18 de manière à présenter son petit côté 26 verticalement ou horizontalement selon le cas. La figure 2c montre en vue de dessus la situation d'examen de la figure 1.

Les mécanismes de translations verticales et horizontales prennent place dans un espace 27 situé entre le bâti 1 (et donc les couronnes 3) et les supports 5 et 22. Ils sont de type connu et constitués avec des systèmes de rails et de glissières. Dans une réalisation pratique, on utilise trois platines parallèles pour procurer ces mouvements.

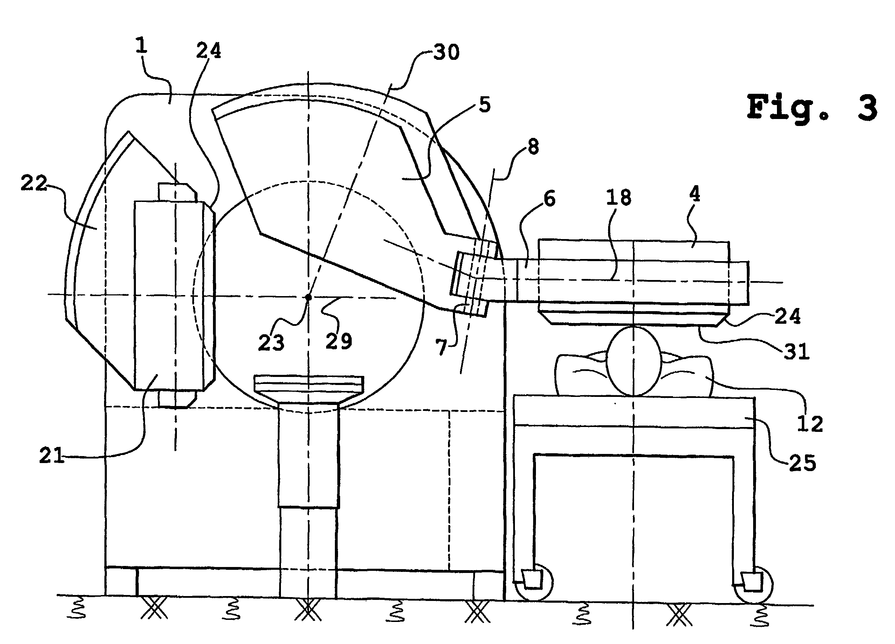

La figure 3 montre, en une vue de face de la machine, une particularité de réalisation de la liaison 7 du rabat 6 à la platine 5. En effet, comme montré sur la figure 1, la position en hauteur du détecteur 4 peut s'avérer inadaptée. En particulier, dans la représentation, le support 5 se trouve en position haute par rapport au bâti 1 du fait de la rotation de la couronne 3, au moment où le rabat 6 est déplacé pour occuper la deuxième position. Normalement la platine 5 a une course en élévation verticale limitée. La limite est telle que le bord inférieur 28 du détecteur 4 ne vienne que légèrement au-dessus d'un plan horizontal 29 passant par l'axe de rotation 23.

On peut aussi utiliser la position basse du support 5. Dans ce but, on ferme le rabat 6 sur le support 5, on le verrouille, et on fait tourner la couronne 3 de 180°. Puis on ouvre à nouveau le rabat 6. Dans ce cas, l'espace utile à l'examen devra être ménagé à gauche du tunnel au lieu d'être ménagé à droite comme ici. Cependant on aboutira à une situation comparable : le bord 28, qui sera alors le bord haut du détecteur 4 dans ce cas, ne viendra que légèrement au-dessous du plan horizontal 29.

Un problème se pose donc si la zone d'examen dans le corps 12 du patient est justement située autour du plan horizontal 29. Pour résoudre ce problème on pourrait prévoir un siège 13 plus haut, ou plus bas. Ce serait également possible pour une machine de travail. Par contre pour un lit d'hôpital, ce n'est pas vraiment possible parce que ceux-ci ne permettent pas, pour des lits simples, un grand débattement en hauteur. Dans ce but on procédera différemment, conformément à la figure 3.

Dans celle-ci, la couronne 3 n'occupe plus une position en rotation correspondant à la présentation horizontale des détecteurs des figures précédentes. Au contraire la couronne 3 aura tourné de quelques degrés. Dans un exemple elle aura tourné de 24° et occupera une direction 30. La direction 30 est une direction contenue dans un plan passant par l'axe 23 et perpendiculaire à la face de détection du détecteur 4 quand celui-ci occupe la première position (celle de la figure 2a). La direction 30 est donc inclinée de 24° par rapport à la verticale. L'axe 8 de l'articulation 7 dans ce mode de réalisation n'est alors pas orthogonal à l'axe 18. Il est au contraire incliné de 12°, la moitié de l'inclinaison de la direction 30, par rapport à l'axe 18. L'axe 18 est ainsi incliné par rapport à un plan perpendiculaire à une face de détection du détecteur 4. Il n'est pas parallèle à un tel plan. En outre le rabat 6 est monté dans l'articulation 7 en étant lui également incliné d'un angle égal de 12°. Autrement dit, lorsque le rabat 6 est déplié, il forme un angle d'environ 156° par rapport à son orientation lorsqu'il est rabattu. Du fait de l'inclinaison de la direction 30 et du décalage latéral de l'articulation 7 sur la platine 5, cette articulation 7 vient se placer en contrebas. Ce placement en contrebas permet alors de franchir suffisamment vers le bas l'altitude de l'horizontale 29, de manière à ce que dans la position dépliée, le détecteur 4 puisse être amené suffisamment bas, près du corps 12 d'un patient couché sur son lit d'hôpital 25. Les valeurs de 12° et 24° sont des solutions préférées de l'invention. Il est toutefois possible de réaliser une telle inclinaison qui ne soit pas égale à 12° (et 24°) mais seulement de l'ordre de 12° (et 24°), par exemple comprise entre 9° et 15° (et 18° et 24°).

Compte tenu du poids présenté par le détecteur 4, de l'ordre de 400 kilos il n'est pas envisageable dans la position horizontale de ce détecteur 4 de le déplacer à la main. Dans ce cas on procédera de la façon suivante. Alors que le rabat 6 est rabattu sur la platine 5, on amène par la rotation de la couronne 3, l'axe 8 à occuper une position verticale. Dans ce cas l'axe 8 est placé en haut de la machine. On déverrouille alors le blocage du rabat 6 sur la platine 5. On tourne ensuite le rabat 6 à la main, ou avec une motorisation légère. Lorsque le rabat 6 occupe la position dépliée, on le verrouille dans cette position, et on complète la rotation de la couronne 3 pour amener le détecteur 4 à l'horizontale. Dans ce cas, la couronne 3 subit deux rotations de 12°, une première pour orienter l'axe 8 verticalement, une seconde pour placer le détecteur 4 horizontalement.

## Revendications

1. Gamma-caméra (1) de type tunnel avec au moins une couronne (3) tournant autour d'un axe (23) de rotation passant dans un tunnel (2), cette couronne portant un détecteur (4) par l'intermédiaire d'un support (5), caractérisée en ce que ce support comporte un rabat (6) lié mécaniquement (7) d'une part à la couronne et d'autre part (15) au détecteur, ce rabat étant muni d'une articulation avec un axe (8) décalé par rapport au détecteur et pouvant occuper au moins deux positions angulaires stables par rapport à cet axe décalé, une première position angulaire permettant de présenter le détecteur qu'il porte en regard du tunnel, une deuxième position angulaire permettant de le présenter à côté du tunnel.

2. Gamma-caméra selon la revendication 1, caractérisée en ce que l'axe décalé est incliné par rapport à un plan perpendiculaire à une face de détection du détecteur.

3. Gamma-caméra selon la revendication 2, caractérisée en ce que l'inclinaison est de l'ordre de 12° d'angle.

4. Gamma-caméra selon l'une des revendications 1 à 3, caractérisée en ce que le détecteur possède un axe d'orientation (18) orthogonal à l'axe de rotation.

5. Gamma-caméra selon la revendication 4, caractérisée en ce que l'axe d'orientation est parallèle à un grand côté de la face de détection du détecteur.

6. Gamma-caméra selon l'une des revendications 4 à 5, caractérisée en ce que le support possède un étrier (15) avec une profondeur plus grande que la moitié d'un petit côté (19) de la face de détection du détecteur.

7. Gamma-caméra selon l'une des revendications 1 à 6, caractérisée en ce que le support comporte une platine (5).

## Patentansprüche

1. Gammastrahlen-Kamera (I) von der Art Tunnel mit mindestens einem um eine einen Tunnel (2) durchquerende Drehachse (23) drehenden Ring (3), der einen Detektor (4) mittels eines Trägers (5) trägt, dadurch gekennzeichnet, daß dieser Träger einen umklappbaren Arm (6) aufweist, der mechanisch (7) einerseits mit dem Ring und andererseits ( 15) mit dem Detektor verbunden ist, wobei dieser umklappbare Arm mit einem Gelenk mit einer in bezug auf den Detektor verschobenen Achse (8) versehen ist und in bezug auf diese verschobene Achse mindestens zwei feste Winkelstellungen einnehmen kann, eine erste Winkelstellung, die es ermöglicht, den von ihm getragenen Detektor vor dem Tunnel anzuordnen, und eine zweite Winkelstellung, die es ermöglicht, ihn neben dem Tunnel anzuordnen.

2. Gammastrahlen-Kamera nach Anspruch 1, dadurch gekennzeichnet, daß die verschobene Achse in bezug auf eine Ebene senkrecht zu einer Erfassungsfläche des Detektors geneigt ist.

3. Gammastrahlen-Kamera nach Anspruch 2, dadurch gekennzeichnet, daß die Neigung einen Winkel von etwa 12° hat.

4. Gammastrahlen-Kamera nach einem der Ansprüche 1 bis 3, dadurch gekennzeichnet, daß der Detektor eine Ausrichtungsachse (18) senkrecht zur Drehachse aufweist.

5. Gammastrahlen-Kamera nach Anspruch 4, dadurch gekennzeichnet, daß die Ausrichtungsachse parallel zu einer großen Seite der Erfassungsfläche des Detektors liegt.

6. Gammastrahlen-Kamera nach einem der Ansprüche 4 bis 5, dadurch gekennzeichnet, daß der Träger einen Bügel (15) aufweist, dessen Tiefe größer ist als die Hälfte einer kleinen Seite (19) der Erfassungsfläche des Detektors.

7. Gammastrahlen-Kamera nach einem der Ansprüche 1 bis 6, dadurch gekennzeichnet, daß der Träger eine Tragplatte (5) aufweist.

## Claims

1. Gamma camera (1) of tunnel type with at least one ring (3) turning about a rotational axis (23) passing into a tunnel (2) ,this ring carrying a detector (4) through the intermediary of a support (5), characterised in that this support comprises a folding arm (6) linked mechanically (7) firstly to the ring and secondly (15) to the detector, this folding arm being provided with an articulation with an axis (8) offset in relation to the detector and capable of occupying at least two stable angular positions in relation to this offset axis, a first angular position making it possible to present the detector which it carries facing the tunnel, a second angular position making it possible to present it beside the tunnel.

2. Gamma camera according to claim 1, characterised in that the offset axis is inclined in relation to a plane perpendicular to a detection face of the detector.

3. Gamma camera. according to claim 2, characterised in that the inclination is of the order of an angle of 12°.

4. Gamma camera according to one of claims 1 to 3, characterised in that the detector has an axis of orientation (18) at right angles to the rotational axis.

5. Gamma camera according to claim 4, characterised in that the axis of orientation is parallel to one large side of the detection face of the detector.

6. Gamma camera according to one of claims 4 to 5, characterised in that the support bas a stirrup (15) with a depth greater than half a small side (19) of the detection face of the detector.

7. Gamma camera according to one of claims 1 to 6, characterised in that the support comprises a plate (5).
